# EUROPÄISCHE PATENTANMELDUNG

(11) **EP 0 764 840 A1**
(43) Veröffentlichungstag der Anmeldung: **26.03.1997**
(21) Anmeldenummer: 96810395.2
(22) Anmeldetag: 14.06.1996
(51) Int. Cl.: G01N 1/20, E04H 4/12, C02F 1/50, G05D 21/02

(54) **Messanordnung für Chlor in Schwimm-/Badebecken**

(30) Priorität: 14.09.1995 EP 95114445
(71) Anmelder: ENDRESS + HAUSER CONDUCTA GESELLSCHAFT FÜR MESS UND REGELTECHNIK mbH & Co., D-70839 Gerlingen (DE)
(72) Erfinder: Gruber, Hans J., Dipl.-Ing., 70825 Korntal-Münchingen (DE)
(74) Vertreter: Morstadt, Volker, Dipl.-Ing. c/o Endress + Hauser Flowtec AG

(57) **Zusammenfassung**

Mit einer Meßanordnung (10) für die Konzentration von Chlor im Wasser (2) von Schwimm/Badebecken (1) von Bädern, insb.von Hallen- oder Freibädern, wobei ein Teil des Wassers dem Schwimm/Badebecken an einer Entnahmestelle (4) zur Aufbereitung entnommen und danach dem Schwimm/ Badebecken (1) als Rückführstrom an einer Zugabestelle (6) wiederzugeführt wird und wobei das Chlor durch Zugabe mindestens eines chlor-haltigen Entkeimungsmittels (7) nach der Aufbereitung dem Rückführstrom zugesetzt wird, sollen auch die Konzentrationswerte des Rückführstroms gemessen werden können. Hierzu enthält die Meßanordnung (10) eine einzige Sensoranordnung (11) für die Konzentration von Chlor, insb. von unterchloriger Säure (HOCl), einen Umschalter (12), über den der Rückführstrom nach Zugabe des Entkeimungsmittels (7) oder über den das dem Schwimm/ Badebecken (1) entnommene Wasser jeweils mindestens teilweise der Sensoranordnung (11) zugeführt ist, und eine Steueranordnung (13) zur periodischen Umschaltung des Umschalters (12).

## Beschreibung

Die Erfindung beschäftigt sich mit einer m³ Meßanordnung für die Konzentration von Chlor und/oder von Chlor-Verbindungen im Wasser von Schwimm/Badebecken von Bädern, insb. von Hallen- oder Freibädern. Schwimm/Badebecken von letzteren enthalten typischerweise zwischen 200 m³ und 600 m³ Wasser, während Badebecken von Krankenhäusern, Rehabilitations-Zentren oder Altenheimen typischerweise zwischen 10 m³ und 200 m³ fassen.

Dieses Wasser wird üblicherweise in einem geschlossenen Kreislauf wiederaufbereitet. Dabei wird ein Teil des Wassers dem Schwimm/Badebecken, insb. kontinuierlich, an einer Entnahmestelle entnommen, aufbereitet und danach dem Schwimm/Badebecken als Rückführstrom an einer Zugabestelle wieder zugeführt. Vor der Aufbereitung kann zeitweise auch Frischwasser in den Kreislauf eingespeist werden. Das Chlor und/oder die Chlor-Verbindung(en) werden bisher durch Zumischung von mindestens einem chlor-haltigen Entkeimungsmittel nach der Aufbereitung dem Rückführstrom zugegeben.

Die Konzentration von Chlor und/oder der Chlor-Verbindung(en) im Wasser des Schwimm/Badebeckens wird bisher an der Entnahmestelle gemessen. Aus der gemessenen Konzentration wird dann die dem Rückführstrom zur Erzielung eines in einer Norm vorgeschriebenen Konzentrationswerts des entnommenen Wassers zuzusetzende Menge des Entkeimungsmittels bestimmt.

Normgemäß vorgeschriebene Konzentrationswerte sind z.B. die in der deutschen Norm DIN 19 643 enthaltenen Werte für freies Chlor im Wasser, nänlich 0,3 mg/l bis 0,6 mg/l an der Entnahmestelle und mindestens 0,3 mg/l im Rückführstrom nach Zugabe in diesen.

Bei dieser Art der Zugabe aufgrund der Konzentrations-Messung an der Entnahmestelle wurde bisher vorausgesetzt, daß der durch die Norm vorgeschriebene Konzentrationswert des Rückführstroms immer gegeben ist. Untersuchungen haben jedoch gezeigt, daß dies meist nicht zutrifft. Daher ist es erforderlich, daß zur Einhaltung der für die Konzentration des Rückführstroms vorgeschriebenen Normwerte auch dessen Konzentrationswerte gemessen werden.

In der NL-A 79 02 177 ist eine Meßanordnung für die Konzentration von Chlor im Wasser von Schwimm/Badebecken von Bädern beschrieben,
- wobei ein Teil des Wassers dem Schwimm/Badebecken an einer Entnahmestelle zur Aufbereitung entnommen und danach dem Schwimm/Badebecken als Rückführstrom an einer Zugabestelle wieder zugeführt wird und
- wobei das Chlor nach der Aufbereitung dem Rückführstrom zugesetzt wird,
- welche Meßanordnung enthält:
   -- eine Regeleinheit,
      --- über die der dem Schwimm/Badebecken entnommene Teil des Wassers und der Rückführstrom in einem einstellbaren Mischungsverhältnis zu einem Mischstrom gemischt werden, und
   -- eine einzige Sensoranordnung für die Konzentration des Chlors im Mischstrom.

Die Bestimmung des Chlors in jedem der den Mischstrom bildenden Eingangsströme, also dem im Schwimm/Badebecken entnommenen Teil des Wassers bzw. im Rückführstrom, ist bei dieser vorbeschriebenen Meßanordnung nur durch Berechnung und Gewichtung aus dem jeweiligen eingestellten Mischungsverhältnis möglich. Dies ist aber umständlich und erlaubt nicht, den momentanen Chlorgehalt sofort zu erhalten und z.B. anzuzeigen.

Die im Patentanspruch definierte Erfindung dient der Behebung dieses Nachteils.

Im Gegensatz zur erwähnten NL-A 79 02 177 schafft die Erfindung somit eine Meßanordnung, die eine zweikanalige Messung intermittierend ermöglicht, wobei die beiden Meßkanäle völlig unabhängig voneinander sind und somit der jeweilige momentane Meßwert sofort zur Verfügung steht. Die an sich vorhandene Sensoranordnung wird entweder zur alleinigen Chlor-Konzentrationsmessung des Schwimm/ Badebecken-Wassers oder zur alleinigen Messung im Rückführstrom herangezogen. Zwei Sensoranordnungen sind nicht erforderlich. Der geringe Mehraufwand für den Umschalter und dessen Steueranordnung ist akzeptabel.

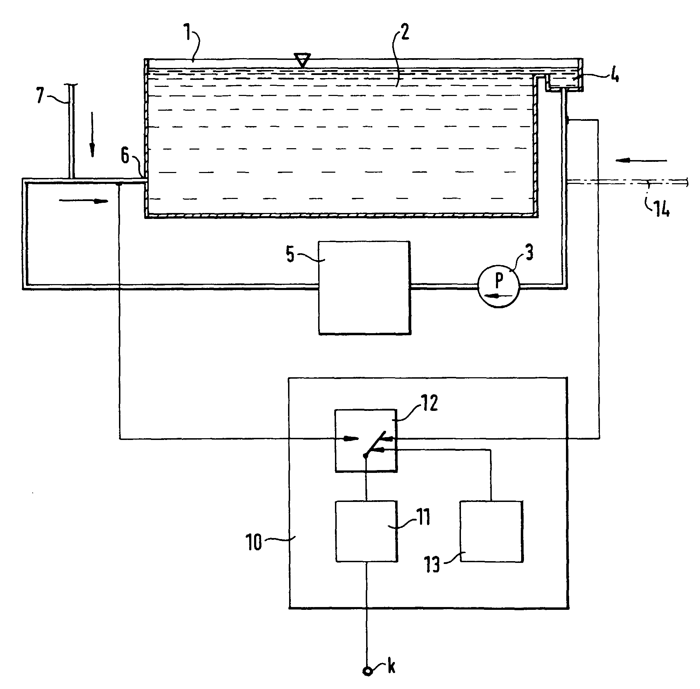

Ein Ausführungsbeispiel der Erfindung wird nun anhand der Figur der Zeichnung näher erläutert, die stark schematisiert nach Art eines Blockschaltbilds eine Meßanordnung zusammen mit den für die Messung wesentlichen Teilen eines Schwimm/Badebeckens zeigt.

Ein im Schnitt gezeigtes Schwimm/Badebecken 1 ist mit Wasser 2 gefüllt, das aus Hygienegründen Chlor und/oder eine Chlor-Verbindung bzw. Chlor-Verbindung(en), insb. freies Chlor, in vorgegebener Konzentration enthalten soll. Die Menge des Wassers 2 bestimmt sich nach dem eingangs genannten Anwendungsfall und liegt somit typischerweise zwischen 10 m³ und 400 m³. Das Wasser 2 wird in einem geschlossenen Kreislauf wieder aufbereitet, wobei eine Umwälzpumpe 3 den Kreislauf unterhält.

Ein Teil des Wassers 2 wird dem Schwimm/Badebecken 1 an einer Entnahmestelle 4, insb. kontinuierlich, entnommen und gelangt in eine Aufbereitungsstufe 5 und von dieser an einer Zugabestelle 6 als Rückführstrom zurück ins Schwimm/Badebecken 1. Das Chlor und/oder die Chlor-Verbindung(en) werden nach der Aufbereitung dem Rückführstrom durch Zumischung von mindestens einem chlor-haltigen Entkeimungsmittel 7 zugegeben, bevor der Rückführstrom an der Zugabestelle 6 ins Schwimm/Badebecken 1 gelangt. Als chlor-haltige Entkeimungsmittel dienen z.B. Cl₂, NaOCl, oder Ca(OCl)₂.

Die Meßanordnung 10 der Erfindung enthält eine einzige Sensoranordnung 11 für die Konzentration von Chlor bzw. der Chlor-Verbindung(en) in Wasser. Die Sensoranordnung 11 ist bevorzugt eine für unterchlorige Säure (HOCl). Ferner enthält die Meßanordnung 10 einen Umschalter 12, z.B. ein Zweiwegeventil, über den bzw. das der Rückführstrom nach Zugabe des Entkeimungsmittels 7 kurz vor der Zugabestelle 6, oder das Wasser aus dem Schwimm/Badebecken jeweils mindestens teilweise, also z.B. ein Teilstrom davon, der Sensoranordnung 11 zugeführt ist. Eine Steueranordnung 13 schaltet den Umschalter 12 periodisch hin und her.

An einem Ausgang der Sensoranordnung 11 entsteht somit ein Ausgangssignal k, das entweder der Konzentration im Rückführstrom oder der Konzentration in dem dem Schwimm/Badebecken 1 entnommenen Wasser proportional ist. Der Ausgang der Sensoranordnung 11 ist zugleich der Ausgang der Meßanordnung 10.

Das Ausgangssignal k kann z.B. auf einem geeigneten Display angezeigt oder mittels eines entsprechenden Recorders auch laufend registriert werden. Ferner ist es möglich, das Ausgangssignal k als Ist-Wert in einem Regelkreis zu verwenden, mit dem ein von einer Norm vorgeschriebener erster Konzentrationswert an freiem Chlor im Wasser 2 des Schwimm/Badebeckens 1 und ein vorgeschriebener zweiter Konzentrationswert an freiem Chlor im Rückführstrom vor der Zugabestelle 6 als Soll-Werte von selbst eingehalten werden.

Wie dies in der Figur gestrichelt dargestellt ist, kann nach Entnahme des dem Umschalter 12 zuzuführenden Anteils an Wasser dem zur Pumpe 3 und zur Aufbereitungsstufe führenden Wasser zeitweise auch Frischwasser 14, z.B. in einem Zwischenbecken, zugesetzt werden.

## Patentansprüche

1. Meßanordnung (10) für die Konzentration von Chlor und/oder mindestens einer Chlor-Verbindung im Wasser (2) von Schwimm/Badebecken (1) von Bädern, insb. von Hallen- oder Freibädern,
- wobei ein Teil des Wassers dem Schwimm/Badebecken an einer Entnahmestelle (4) zur Aufbereitung entnommen und danach dem Schwimm/Badebecken (1) als Rückführstrom an einer Zugabestelle (6) wieder zugeführt wird und
- wobei das Chlor und/oder die Chlor-Verbindung(en) durch Zugabe mindestens eines chlor-haltigen Entkeimungsmittels (7) nach der Aufbereitung dem Rückführstrom zugesetzt wird,
- welche Meßanordnung (10) enthält:
-- eine einzige Sensoranordnung (11) für die Konzentration von Chlor und/oder der Chlor-Verbindung(en), insb. von unterchloriger Säure (HOCl),
-- einen Umschalter (12)
--- über den der Rückführstrom nach Zugabe des Entkeimungsmittels (7) oder über den das dem Schwimm/ Badebecken (1) entnommene Wasser jeweils mindestens teilweise der Sensoranordnung (11) zugeführt ist, und
-- eine Steueranordnung (13) zur periodischen Umschaltung des Umschalters (12).
